# EUROPEAN PATENT APPLICATION

(11) **EP 2 042 183 A1**
(43) Date of publication of application: **01.04.2009**
(21) Application number: 07019026.9
(22) Date of filing: 27.09.2007
(51) Int. Cl.: A61K 36/47, A61K 36/484, A61K 36/42

(54) **Herbal compositions**

(71) Applicant: Scindia AG, 6060 Sarnen (CH); Innovative Naturals Inc., Paschim Vihar Dehli 110063 (IN)
(72) Inventor: Krishan, Gobi, Delhi 110063 (IN); Buecker, Rolf Dieter, 87781 Ungerhausen (DE)
(74) Representative: Grimm, Siegfried

(57) **Abstract**

The invention relates to herbal compositions comprising extracts of the plants *Benincasa hispida, Glycyrrhiza glabra* and *Embilica officinalis.* The invention also reveals a method of preparing and using these compositions.

## Description

The present invention relates to herbal compositions, their use in medicine, and manufacturing processes for the disclosed compositions.

Mental disorders comprise a broad range of conditions with different symptoms, including depression and dementia. Dementia is the progressive decline in cognitive function, the most common type being Alzheimer's disease. Studies reveal that in 2005, the worldwide prevalence of dementia was estimated to be about 24.3 million people, with 4.6 million new cases of dementia every year. The number of affected people will double every 20 years. Moreover, about 60% of people with dementia live in developing countries (see e.g. Ferri CP, Prince M, Brayne C, et al (2005). "Global prevalence of dementia: a Delphi consensus study". Lancet 366 (9503): 2112-7).

Furthermore, according to the World Health Organization, mental health problems (excluding mental disorders) account for nearly 20% of the burden of disease in the European Region. One in four people at some time in life are affected by mental health issues.

A further health problem, predominantly in developed countries, is the metabolic syndrome (also called syndrome X), a combination of disorders that increase the risk for cardiovascular disease and diabetes. The disorders include abdominal obesity, atherogenic dyslipidemia, elevated blood pressure and insulin resistance. Some studies estimate the prevalence of syndrome X to be up to 25% of the US population.

Due to the large prevalence, many pharmaceutical companies have oriented their research efforts to the above mentioned diseases. However, synthetic medicaments are often not affordable for poorer countries. Furthermore, many pharmaceuticals have the drawback of major side effects. Also, they often only reduce the symptom without attacking the cause of the disease.

Hence, there is a need for further medicaments/compositions which effectively help to diminish the effects and/or facilitate the cure of the above mentioned diseases and which overcome the above mentioned drawbacks.

It is a general object of the invention to provide a composition based on herbal ingredients.

It is understood that the various embodiments, preferences and ranges may be combined at will. Further, depending of the specific embodiment, selected definitions, embodiments or ranges may not apply.

The following definitions shall apply throughout the entire specification unless otherwise specified:

The terms "fortification" and "fortified" are known in ayurvedic medicine; in particular, they refer to an enrichment process: for example, a juice or extract of any part of a plant is added to a dried powder of said plant part. The mixture may then be concentrated. Optionally, the process may be repeated several times, thereby enriching the product in active ingredients.

Any effective part of a plant in accordance with the present invention can be used, including - without being limited to - fruits, seeds, leaves, stems, flowers, roots, berries, bark, or any other plant parts that are useful for the purposes described. The plants used can be in any form which is effective, including but not limited to dry powders, grounds, emulsions, extracts, and other conventional compositions.

Unless otherwise specified, all indicated percentages refer to weight percentages.

The term "cryogenic conditions" refers to conditions at low temperature which may be caused by the use of dry ice or liquid nitrogen.

The composition of the present invention follows the tradition of Ayurvedic medicine, an ancient system of integral health care, which aims at attacking the cause of a disease. Being made of extracts of several widely available plants, the inventive composition can be prepared economically and easily.

Now, in order to implement these and still further objects of the invention, which will become more readily apparent as the description proceeds, the composition contains (i.e. comprises or consist of) *Emblica officincalis, Benincasa hispida* and *Glycyriihza glabra.* The mixture of the three plants shows a synergistic effect when compared to the effects caused by each plant individually.

It was surprisingly found that the herbal compositions and methods of the present invention augment high density cholesterol (HDL-C), Apolipoprotein A-1 (APO A-1), Apo A-1 /Apo B ratio and reduce Apolipoprotein B(APO B), low density cholesterol (LDL-C), triglycerides (TG), very low density cholesterol (VLDL), lipoprotein (a) and Apo B /APO A-1 ratio. Thereby, the inventive composition facilitates and/or promotes longevity, cognition and reduces ageing , senescence, liver failure, depression, dementia, Alzheimer, degenerative brain disorders and parameters responsible for syndrome X (metabolic syndrome), Insulin resistance, and/or CETP (cholesteryl ester transfer protein). Furthermore, the composition and methods of the present invention are useful for lowering the blood levels of circulating inflammatory cytokines such as IL-6, TNF alpha etc. and can be helpful in regulating low and or raised serum cholesterol levels. The composition also may be used for augmenting low birth weight of fetuses.

It is believed that the compositions of the present invention remove the cause of a symptom, not only the symptom itself.

In an advantageous embodiment, the composition may additionally contain sugar, preferably a sugar Khand variety, clarified butter and/or *Crocus sativus.*

In a further advantageous embodiment, the compostion may also contain extracts of *Tribulus terrestris, Tinospora cordifolia, Panax ginseng, Withania somnifera* and/ or *Achyranthus aspera.*

The composition contains 5 to 40% per weight of *Emblica officincalis* (amla), preferably 10 to 20 % w/w, more preferably 20 % w/w. Amla can be used as commercially available, however, in a preferred embodiment, a fortified amla concentrate can be manufactured as follows: The extract of the amla fruit in 90 to 95 volume % of alcohol (6:1) is dried. The dried extract is fortified with 25 times its volume with fresh amla fruit juice which is already filtered and/or clarified. The mix is then fortified by performing a rotary vacuum extraction process or a thin film extraction process. The fortification may be repeated several times, preferably up to 50 times, more preferably up to 100 times, each time adding the fresh amla juice to dried product and re-extracting the mix. The resultant fortified product is vacuum dried and used for formulation.

*Emblica officinalis* can be used as an extract containing up to 50 % polyphenols, more than 50% of tannins and about 5% of vitamin C using dried amla powder/extract prepared in any solvent of any percentage with further fortification with raw amla fruit juice any number of magnitude over its dry powder, extract or solvent extractatives as explained.

Furthermore, the inventive composition contains juice of ripe or raw fruits of *Benincasa hispida* (wax gourd), at a minimum concentration of 28% per weight of the final composition. The juice is usually freshly prepared.

Moreover, the composition contains *Glycyriihza glabra* (liquorice), preferably in a concentration of 5-10% per weight of the final composition, more preferably 7 to 9% w/w. From *Glycyriihza glabra,* preferably extracts of the peeled root are used. The root contains glyrcyrrhizin, a yellow amorphous powder, which is preferably extracted by a supercritical fluid extraction process. The extract preferably contains 40 % w/w of glyrcyrrhizin. Such extracts are commercially available, e.g. of Nisarga (India).

In one embodiment, clarified butter (Ghee) may be added. One effect of this addition is that the viscosity of the mixture will be enhanced. It is particularly beneficial to include an effective amount of clarified butter in a formulation intended for mental health or neurodegenerative disorders. The term "clarified butter" refers to unsalted butter which was melted and milk solids and water were removed by density separation. Thus, essentially liquid butterfat remains. Said clarified butter is also known as "ghee". The clarified butter is preferably aged; more preferably, the clarified butter has an age of about 1 ½ years. The amount to be added is to complete the balance to 100%.

Furthermore, sugar may be added at a concentration of up to 25% per weight, more preferably 18 to 20% per w/w. Preferably, a variety of sugar Khand (crystallized lumps of raw sugar) is used.

In an advantageous embodiment, the compositions may contain 1 to 3 % w/w of dried stigma of *Crocus sativus* (saffron). Preferably, Baby® Saffron from Kashmir (USMS Saffron Co. Inc., New Delhi, India) is used.

In a further embodiment, the composition additionally contain 4 to 8%, preferably 7-8%, of one or more of the extracts of plants selected of the group consisting of *Tribulus terrestris, Tinospora cordifolia, Panax ginseng, Withania somnifera* and *Achyranthus aspera.*

Preferably, aqueous extracts of the dried fruits of *Tribulus terrestris* (caltrop) are used (e.g. from Ansar Industries, (Surat, India)). The *Tribulus terrestris* extract may comprise up to 40% of saponins.

Of *Tinospora cordifolia* (guduci) 10:1 aqueous extracts of all five parts of the plant, including root, stem, leaves, flower and fruits are preferably used (obtainable from Ansar Industries, Surat, India). Said five parts of a plant are referred to as "pachang" in ayurvedic medicine. The concentration is preferably 10-20%.

Any effective species and/or variety of ginseng can be used, e.g. *Panax ginseng,* Korean or Chinese ginseng. Preferably, *Panax ginseng* (e.g. available from Helioziang Province of China; purchasable from different suppliers) is used. Preferably, extracts of the root are used.

The inventive composition contains preferably water:methanol extracts of the root, preferably 10:1 extracts, of *Withania somnifera* (winter cherry, also called Indian ginseng). Such an extract is e.g. obtainable from Garlico Herbals (Mandsaur, India).

Water:methanol extracts of "pachang" (root, the stem, the leaves, flower and fruits), preferably 10:1 extracts, of *Achyranthus aspera* (devil's horsewhip) are used in a preferred embodiment. Such an extract is e.g. obtainable from Garlico Herbals (Mandsaur, India).

Extraction methods useful in the context of this invention are known to the skilled person. For example, to extract or concentrate the effective ingredients of a plant, the plant part is typically contacted with a suitable solvent, such as water, an alcohol (e.g. methanol or ethanol), or any other solvents, or mixed solvents. The choice of the solvent can be made routinely e.g. based on the properties of the active ingredient that is to be extracted or concentrated by the solvent.

Preferably, extracts of the plants are used, e.g. 20:1, 10:1, 7:1 and/or 5:1 extract. The ratios indicate that the dry weight of the herb is concentrated e.g. ten times (10:1), seven times (7:1) and/or five times (5:1) in the extract. Furthermore, the plants may be extractable in any other solvent. Also, any ratio, e.g. an aqueous extract / alcohol extract, may be used.

The aforementioned formulas can be prepared with any ratio of herb, extract, and any of the aforementioned values can refer to the weight of the extract.

The ingredients of the composition may be combined in "synergistic amounts." By this it is meant that the amounts of the ingredient present in the composition (and in combination with the other ingredients) is more effective at achieving the desired purpose than when administered alone or in another herbal composition. Synergy indicates that the ingredients cooperate with each other to achieve an enhanced or superior result than would be achieved if either were administered alone, i.e. the effect is not additive (the sum is greater than the sum of the individual parts). Thus, the invention relates to a synergistic composition, comprising the compounds as described herein.

The compositions can be used in any effective formulation including oral formulations such as pills, capsules, troche, liquid, extract, beverage, food, tea; topical formulations, such as cream, spray, gel; injectable formulations, etc. Thus, the invention also relates to such formulations, comprising the compounds as described herein.

The composition of the present invention may further contain inert and carrier ingredients, stabilizers, antioxidants, etc., including, but not limited to water, salt solutions, alcohols, gum arabic, vegetable oils, benzyl alcohols, gelatin, carbohydrates such as lactose, amylose or starch, magnesium stearate, talc, silicic acid, viscous paraffin, perfume oil, fatty acid monoglycerides and diglycerides, pentaerythritol fatty acid esters, hydroxy methylcellulose and the like. Other additives include e.g. antioxidants and preservatives, coloring agents, flavoring agents and diluting agents, emulsifying agents and suspending agents, such as acacia, agar, alginic acid, sodium alginate, bentonite, carbomer, carrageenan, carboxymethylcellulose, cellulose, cholesterol, gelatin, hydroxyethyl cellulose, hydroxppropyl cellulose, hydroxypropyl methylcellulose, methylcellulose, octoxynol 9, oleyl alcohol, povidone, propylene glycol monostearate, sodium lauryl sulfate, sorbitan esters, stearyl alcohol, tragacanth, xanthan gum, and derivatives thereof, solvents, and miscellaneous ingredients such as microcrystalline cellulose, citric acid, dextrin, dextrose, liquid glucose, lactic acid, lactose, magnesium chloride, potassium metaphosphate, starch, and the like.

The inventive composition augments biochemical parameters in the blood and/or serum, such as High density cholesterol (HDL-C), Apolipoprotein A-1 (APO A-1), Apo A-1 /Apo B ratio. Furthermore, Apolipoprotein B (APO B), low density cholesterol (LDL-C), Triglycerides (TG), Very low density cholesterol (VLDL), lipoprotein (a) and the Apo B /APO A-1 ratio are reduced.

The invention is not limited by how the augmentation or reduction in the named parameters is effected. Various mechanisms, acting alone or in combination, produce the result as explained. This includes e.g. promoting metabolism, promoting utilization of chemical fuels (e.g. fats and carbohydrates), reducing blood cholesterol levels, acting as diuretic, promoting utilization of stored fuels, such as fat from adipose tissue and glycogen.

The physiological effects of the above described composition include, without being limited to, longevity, cognition; reduction of ageing , senescence, liver failure, depression, dementia, Alzheimer, degenerative brain disorders and parameters responsible for syndrome X (metabolic syndrome), Insulin resistance, and/or CETP (cholesteryl ester transfer protein). Furthermore, the composition and methods of the present invention are useful for lowering the blood levels of circulating inflammatory cytokines such as IL-6, TNF alpha etc. and can be helpful in regulating low and or raised serum cholesterol levels. The composition also may be used for augmenting low birth weight of fetuses.

The compositions can be administered alone, or in combination with other active and/or inert agent(s).

The composition of the present invention may be administered in an effective amount to a subject in need thereof. By the term "administered" it is meant that the composition is delivered to a subject by any means or route which is effective to achieve the desired result, including, e.g., oral, parenteral, enteral, intraperitoneal, topical, transdermal (e.g., using any standard patch), ophthalmic, nasally, local, non-oral, such as aerosal, inhalation, subcutaneous, intravenous, intramuscular, buccal, sublingual, rectal, vaginal, intra-arterial, intrathecal administration.

An "effective amount" relates to the indication that the compositions and methods are useful to encourage, elicit, cause and/or produce the biochemical and/or physiological effects indicated above. The invention is not limited by how much blood / serum parameters are promoted or facilitated. Other parameters influencing the "effective amount" include diet, exercise and hereditary aspects.

Compositions may be administered at any suitable time. For example, compositions can be administered before, after or together with an activity, e.g. prior to or after a meal, an exercise, a sporting event, etc. Times of administration before the activity can vary to be effective e.g. about 6 hours, 4 hours, 2 hours, 1.5 hours, 1 hour, 30 minutes, 15 min, 10 min, 5 min, etc. Additionally, the compositions may be administered concurrently with the activity. The composition of the invention may also be administered when nutrients from a meal or snack begin to appear in the blood, i.e. the absorptive state, when ingested nutrients are entering the blood from the gastrointestinal tract (see e.g. Human Physiology, Vander et al., Fifth Edition, 1990, e.g., Chapters 16 and 17). The adsorptive state can vary, depending upon the content of the food e.g. from about 1 hour to about 4 hours.

In a further aspect, the invention reveals the use of the inventive composition for the treatment, prevention, delay of progression of a disorder selected from the group of insulin resistance, metabolic disorders, depression, Alzheimer, degenerative brain disorders and/or syndrome X.

In another aspect, the invention discloses a medicament containing the composition described herein for the treatment, prevention, delay of progression of a disorder selected from the group of insulin resistance, metabolic disorders, depression, Alzheimer, degenerative brain disorders and/or syndrome-X.

The composition may further be used for the manufacture of a medicament for the treatment, prevention, delay of progression of a disorder selected from the group of insulin resistance, metabolic disorders, depression, Alzheimer, degenerative brain disorders and/or syndrome X.

In still another aspect, the present invention relates to a process for manufacturing the compositions described herein. The process comprises the steps of:
a) Providing an extract of *Glycyrhizza glabra;*
b) Optionally adding clarified butter to the extract of step a) to form a paste;
c) Adding to the extract of a) to the paste of b) juice of *Benincasa hispida* to fortify the mixture;
f) Concentrating the mixture;
g) Adding a fortified extract of *Embilica officinalis* to the mixture of step e); and
h) Mixing under cryogenic conditions.

For step a), an extract, with up to 40 % of active constituent of *Glycyrhizza glabra,* i.e. glycyrhizzin, is provided. Said extract is preferably produced by a supercritical fluid extraction process.

In step b), the extract is preferably mixed with clarified butter of any quantity so as to form a paste. Preferably, the clarified butter constitutes 5 to 10 % of the final composition.

The resulting paste of step b) or the extract of a) is fortified with up to 120 times or more clarified and/or centrifuged juice, preferably fresh juice, of *Benincasa hispida.* Typically, 10 to 15 liters of juice are used for 1 kg of the final product.

In step f), the mixture is concentrated, preferably by gently heating the mixture in a vessel for up to the time the entire mixture is devoid of water. Alternatively, this step may be done in a rotary vacuum extractor and/or a thin film evaporation equipment. The final humidity of the paste should be around 4%.

Then, a fortified extract of *Embilica officinalis* as described above is added.

In a further step, both compositions are mixed.

In an alternative embodiment, one or more extracts selected of the group consisting of: extracts of *Achyranthus aspera, Tinospora cordifolia, Withania sommnifera, Panax ginseng* and/or *Tinospora cordifolia* are added to the inventive composition by mixing under cryogenic conditions. Preferred concentrations of extracts and the relevant plant parts are disclosed above. Preferably, the extracts are provided in dried form.

The mixing is completed in cryogenic conditions, e.g. using dried ice, and/or liquid nitrogen.

In a preferred embodiment, the composition contains dried stigma of *Crocus sativus* and/or sugar, preferably sugar khand. Both ingredients are added prior to processing in cryogenic conditions.

The present invention further encompasses a composition obtained according to the process described herein.

In another aspect, the invention discloses a medicament comprising the composition described herein for the treatment, prevention, or delay of progression of a disorder as described herein.

In still another aspect, the composition described herein may be used for the manufacture of a medicament for the treatment, prevention, or delay of progression of a disorder as described herein.

In another aspect, the invention discloses a method of treatment, comprising the step of administering to a subject in need thereof with an effective amount of a composition as described herein for the treatment, prevention, or delay of progression of a disorder as described herein.

While there are shown and described presently preferred embodiments of the invention, it is to be distinctly understood that the invention is not limited thereto but may be otherwise variously embodied and practiced within the scope of the following claims. The following examples further illustrate the invention.

### Example 1: Preparation of a herbal composition

A herbal composition is prepared containing, by weight, 28% of freshly prepared *Benincasa* extract*, 8% of 40% *Glycyrrhiza glabra* extract (Nisarga Biotech, India), made with 4% of clarified butter (ghee made from cow milk), 20 % of amla material made by fortification of its 95% alcohol extract with twenty five times quantity of pure clarified juice of amla fruits by rotary vacuum extraction process, 8% of each of extracts of 10% *Panax ginseng* (from the Heliozhang province of China), 40% *Tribulus terrestris* (of Ansar Industries, Surat, India), 10:1 extracts of *Tinospora cordifolia* (of Ansar Industries, Surat, India). Further, *Achyranthus aspera* 10: 1 extract (Garlico Herbals, Mandsaur, India), 6 % of 10:1 of *Withania somnifera* extract of Garlico Herbals, Mandsaur, India and 2% *Crocus sativus* (USMS Saffron Co. Inc., New Delhi, India), *and* 20% by weight of sugar (khand) was prepared. The mixing of all constituents was done in cryogenic conditions using dry ice.

*Such extract is prepared from centrifuged / clarified juice of ripened raw fruit. About 10-15 litres of juice is used to make 1 kg of product which corresponds to 28% of its contribution to the final product.

### Example 2: Clinical tests

A clinical trial was performed using MFM "sample no.1", the herbal composition prepared in Example No. 1. A clinical trial on eight persons was performed with one person on placebo. All persons took 6 to 8 grams of the "sample no 1" in two divided doses with water in morning and evening times. Patients in the control group took four placebo capsules (800 mg/capsule) each time, twice a day. All six persons took their respective formulation for 30 to 60 days. Clinical tests were performed following standard protocols on day zero and after 15 / 30 / 45 / 60 days. All patients were instructed to take sensible diet and do mild to moderate exercise & take their usual day to day required regular medication for blood pressure / diabetes etc.

The effect on augmentation on HDL-C Apo A-1, ratio of Apo A-1 / Apo B was from 10 to 70 % with Apo B, Triglycrides, VLDL-C, LDL-C and ratio of Apo B / Apo A-1 following the downward trend. The effect on lipoprotein (a) seemed to have a mixed trend but no steep increase in its value was observed. The formulation was well tolerated on liver / kidney and haemotogical profiles with only one case where it showed an upward trend on liver profile only. Table 1 summarizes the results.

In a different sample group, further blood parameters were determined. The results are shown in table 2.

A further sample group received placebo treatment. The results are shown in table 3.

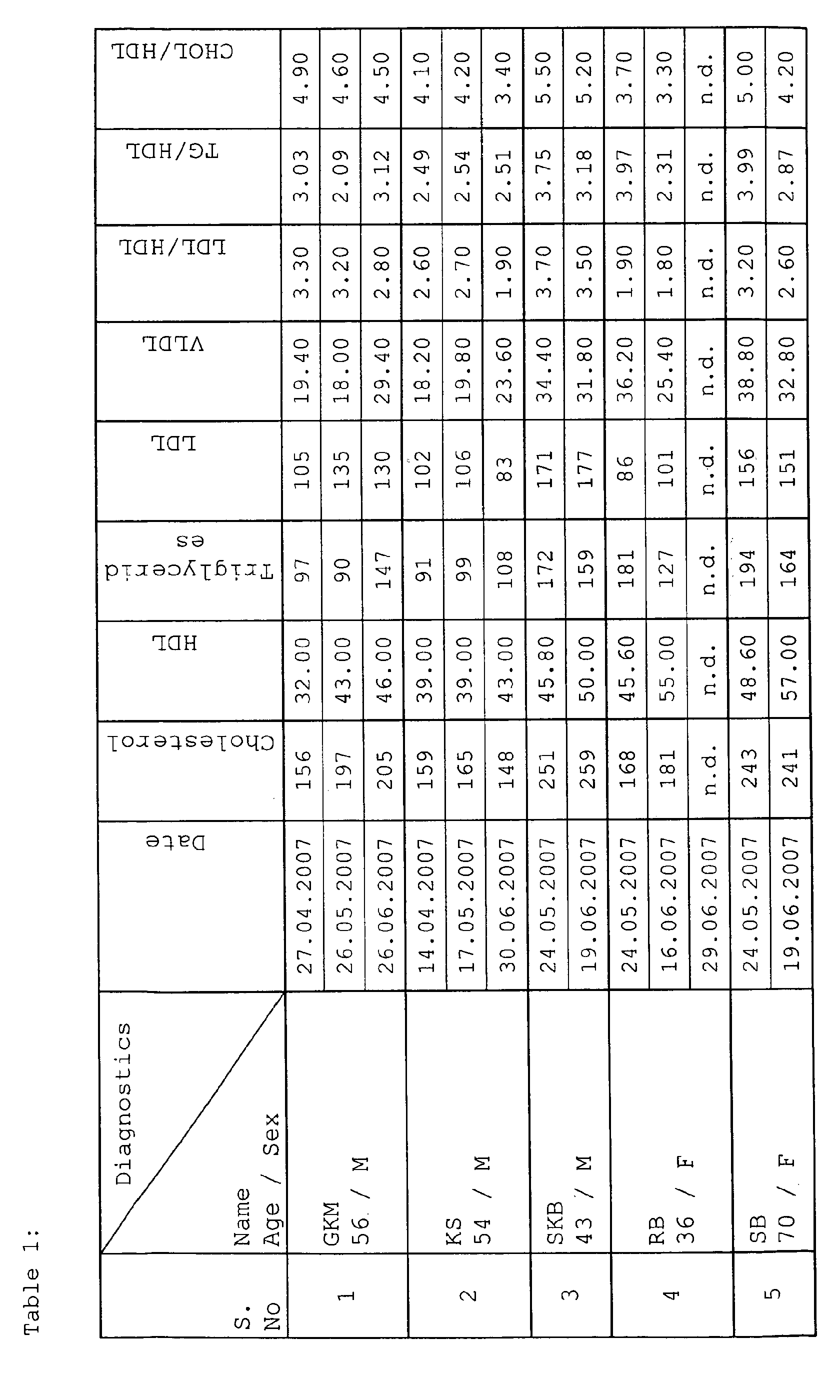

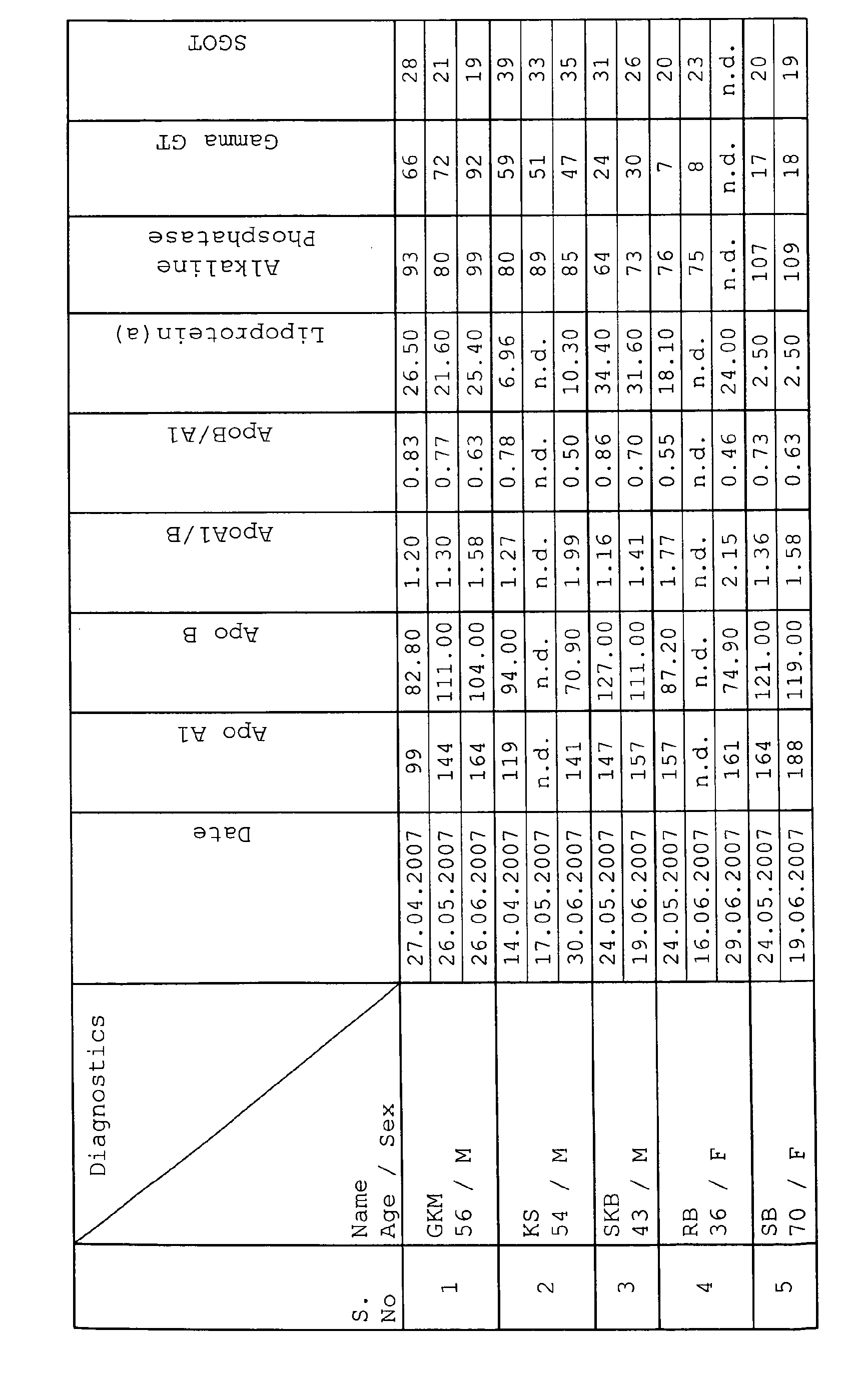

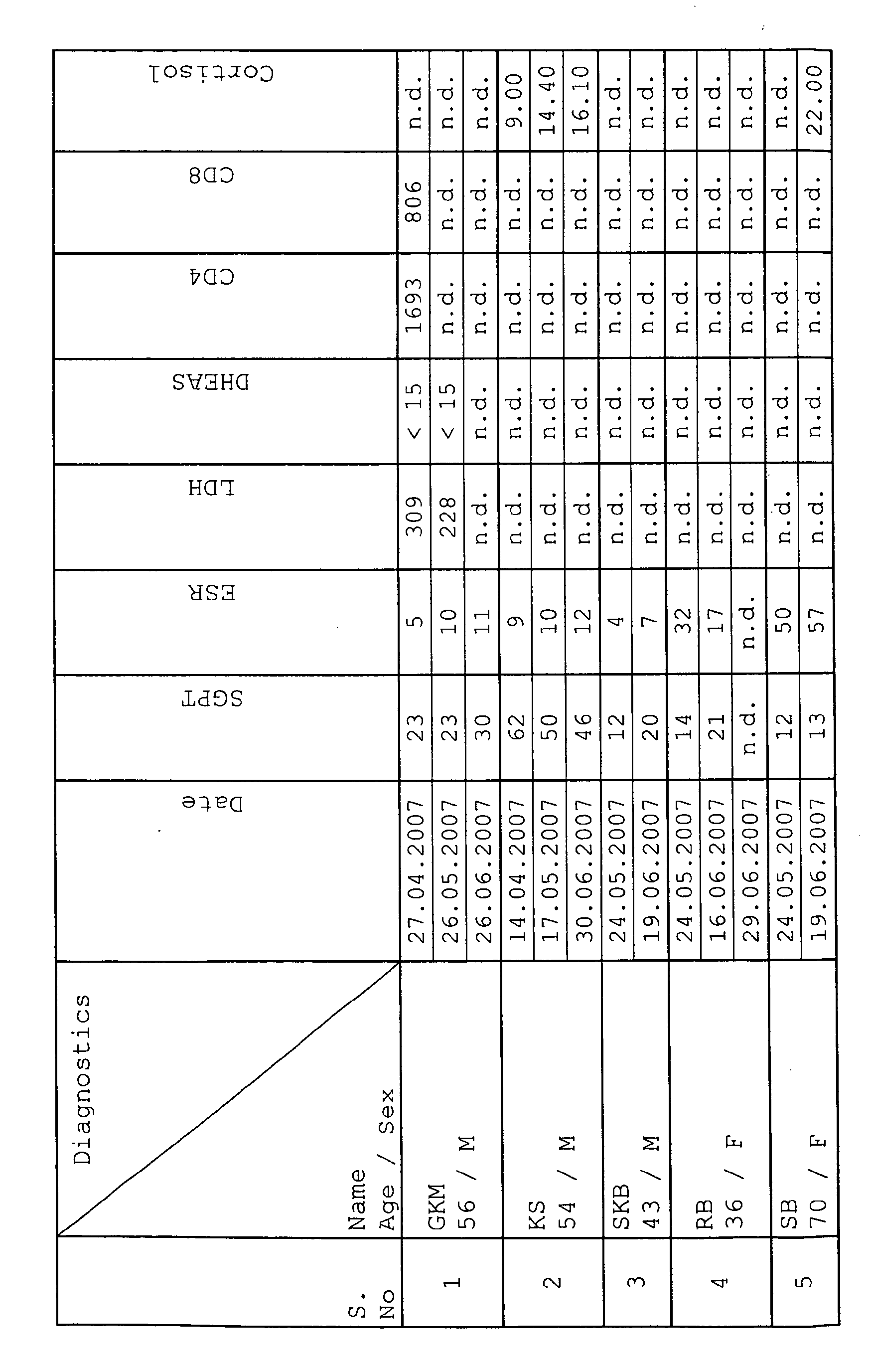

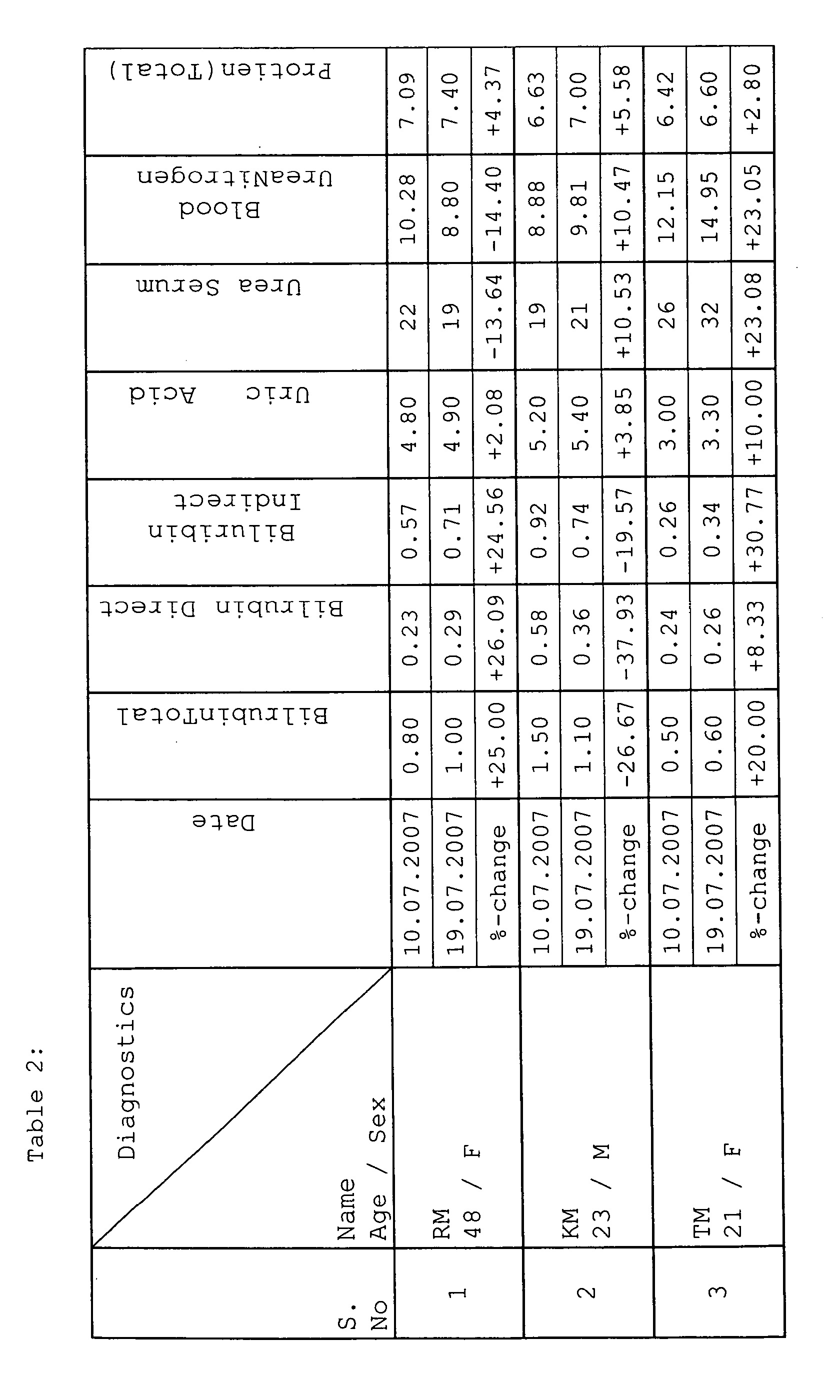

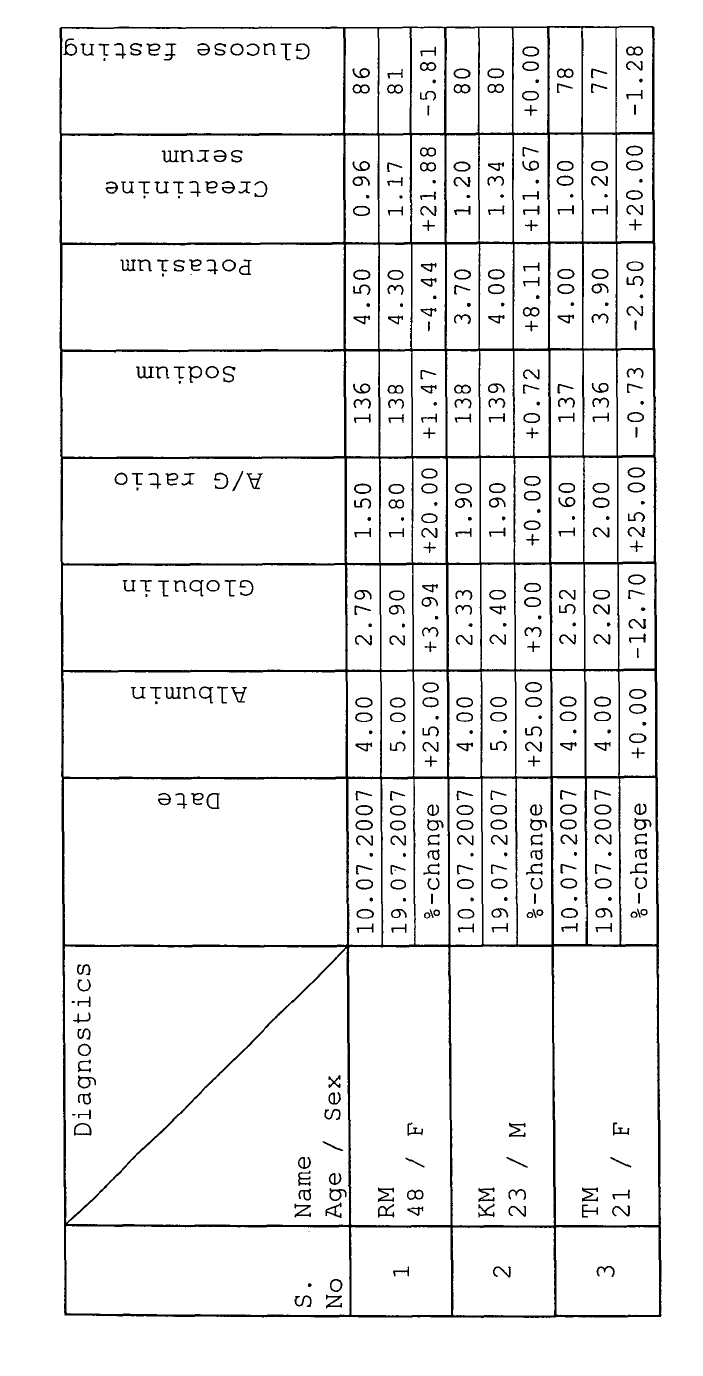

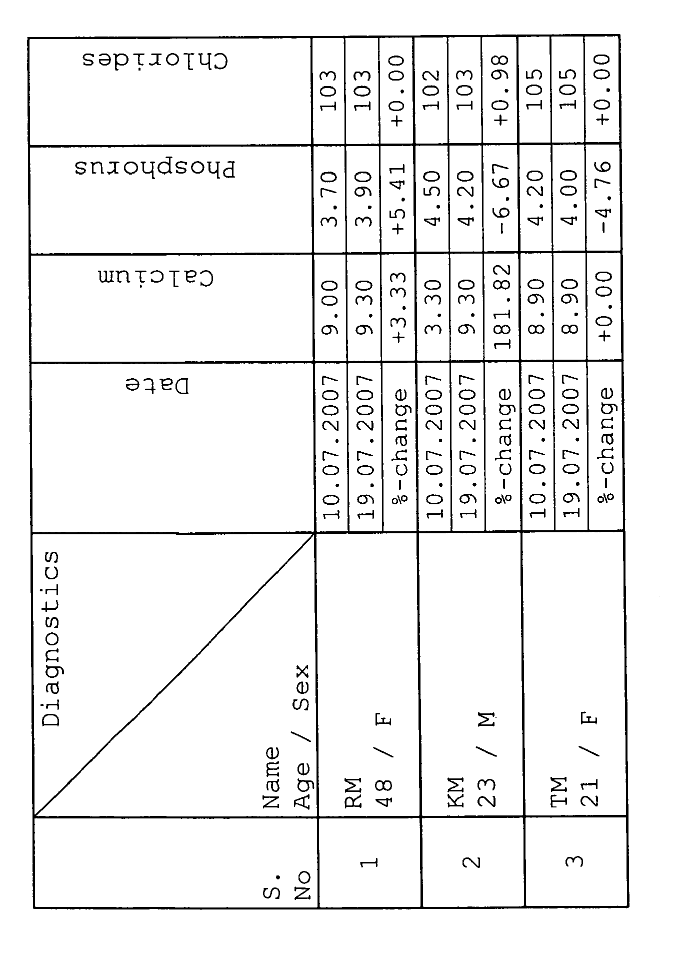

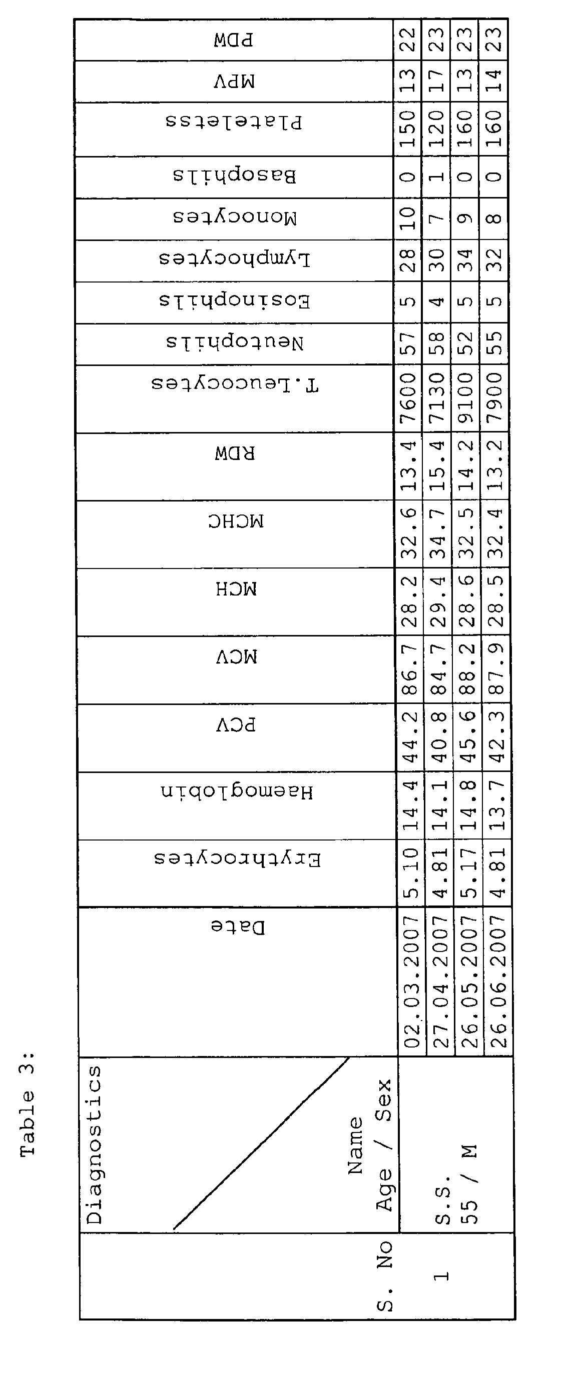

## Claims

1. A composition comprising extracts of *Benincasa hispida, Glycyrrhiza glabra* and *Embilica officinalis.*

2. The composition of claim 1, further comprising one or more components selected of the group consisting of sugar, clarified butter and extracts of *Crocus sativus.*

3. The composition of claim 1 or 2, further comprising one or more extracts selected from the group consisting of extracts of *Achyranthus aspera, Tinospora cordifolia, Withania sommnifera, Panax ginseng* and *Tinospora cordifolia.*

4. The composition of anyone of the preceding claims, comprising 10-20% of *Embilica officinalis.*

5. The composition of anyone of the preceding claims, comprising at least 28% of *Benincasa hispida.*

6. The composition of anyone of the preceding claims, comprising 5 to 8% of *Glycyrrhiza glabra.*

7. The composition of anyone of claims 2 to 6, comprising 1 to 3% of *Crocus sativus.*

8. The composition of anyone of claims 3 to 7, comprising 4 to 8%, more preferably 7 to 8%, of one or more extracts selected from the group consisting of *Achyranthus aspera, Tinospora cordifolia, Withania sommnifera, Panax ginseng* and *Tinospora cordifolia.*

9. The composition of anyone of claims 2 to 8, comprising up to 25% of sugar, preferably of sugar khand.

10. The composition of anyone of the preceding claims, further comprising one or more ingredients selected from the group consisting of: carriers, stabilizers, antioxidants, preservatives, coloring agents, flavoring agents, diluting agents, emulsifying agents and suspending agents.

11. A manufacturing process for the herbal composition according to anyone of claims 1 to 10, comprising the steps of:
a) Providing an extract of *Glycyrhizza glabra*
b) Optionally adding clarified butter to the extract of step a);
c) Fortifying the extract or paste with fresh juice of *Benincasa hispida;*
d) Concentrating the mixture;
e) Adding a fortified extract of *Embilica officinalis* to the mixture of step d); and
f) Mixing under cryogenic conditions.

12. The process of claim 13, further comprising the step of adding *Crocus sativus* and/or sugar, preferably sugar khand, before step f).

13. The process of claim 11 or 12, further comprising the step of adding one or more extracts selected from the group consisting of extracts of *Achyranthus aspera, Tinospora cordifolia, Withania sommnifera, Panax ginseng* and *Tinospora cordifolia* to the mixture.

14. A composition obtained according to the process as described in any of claims 11 to 13.

15. A medicament comprising the composition of anyone of claims 1 to 10, 14 for the treatment, prevention, or delay of progression of a disorder selected from the group of insulin resistance, metabolic disorders, depression, Alzheimer, degenerative brain disorders and/or syndrome X.

16. Use of the composition of anyone of claims 1 to 10, 14, for the manufacture of a medicament for the treatment, prevention, or delay of progression of a disorder selected from the group of insulin resistance, metabolic disorders, depression, Alzheimer, degenerative brain disorders and/or syndrome X.
